# EUROPÄISCHE PATENTSCHRIFT

(11) **EP 2 275 214 B1**
(45) Veröffentlichungstag und Bekanntmachung des Hinweises auf die Patenterteilung: **09.05.2012**
(21) Anmeldenummer: 10168822.4
(22) Anmeldetag: 08.07.2010
(51) Int. Cl.: B08B 3/02, B08B 3/04, B08B 1/00

(54) **Vorrichtung zum Behandeln von Behältnissen mit Trägersterilisation**
Device for handling containers with holder sterilisation
Dispositif de traitement de récipients par stérilisation de support

(30) Priorität: 18.07.2009 DE 102009033809
(43) Veröffentlichungstag der Anmeldung: 19.01.2011
(60) Teilanmeldung: 12150982.2
(73) Patentinhaber: Krones AG, 93073 Neutraubling (DE)
(72) Erfinder: Winzinger, Frank, 93051 Regensburg (DE); Albers, Philipp, 32756 Detmold (DE)
(74) Vertreter: Hannke, Christian

(56) Entgegenhaltungen:
- DE-A1- 4 308 757
- DE-A1-102007 017 938
- DE-A1-102007 048 861
- US-A- 4 375 992

## Beschreibung

Die vorliegende Erfindung bezieht sich auf eine Vorrichtung und ein Verfahren zum Behandeln von Behältnissen. Aus dem Stand der Technik ist es seit längerem bekannt, dass bei der Herstellung von Kunststoffbehältnissen, beispielsweise von Getränkeflaschen, zunächst Kunststoffvorformlinge erzeugt werden, diese anschließend erwärmt und mit Blasumformung zu Behältnissen expandiert werden. Die Erfindung findet insbesondere auf derartige Vorrichtungen Anwendung, es wird jedoch darauf hingewiesen, dass die erfindungsgemäße Vorrichtung auch bei anderen Maschinen beispielsweise solchen Maschinen, welche Glasbehältnisse abfüllen, Anwendung finden.

In dem Bereich der getränkeherstellenden Industrie ist in besonderer Weise auf Reinheit und Keimfreiheit beim Abfüllprozess zu achten. Die unterschiedlichen Behandlungsschritte der Behältnisse bringen es dabei mit sich, dass die herzustellenden und abzufüllenden Behältnisse von einer Vielzahl von Transporteinrichtungen transportiert werden. Dieser Transport kann dabei wiederum zu Verschmutzungen führen.

Aus der WO 2008/125 216 A1 sind eine Behälterherstellungsvorrichtung und ein Herstellungsverfahren für Formkörper bekannt. Bei dieser Vorrichtung wird beschrieben, dass Strahlungseinrichtungen, wie UV-Strahler, beispielsweise auf einem Blasrad angebracht sind und mit diesen UV-Strahlem Teile der Oberflächen der Behälterherstellungsvorrichtung, wie beispielsweise Gehäusewandungen, gereinigt werden. Diese Vorrichtung erlaubt damit eine teilweise zufriedenstellende Sterilisation von Maschinenteilen jedoch stellen namentlich jene Elemente, welche die Behältnisse führen, einen besonderen Risikofaktor hinsichtlich Verkeimung dar. Dies gilt insbesondere für solche Halteelemente, welche zum Transport der Vorformlinge in diese eingreifen oder für solche, welche die Behältnisse an ihren Mündungen greifen.

Der vorliegenden Erfindung liegt daher die Aufgabe zugrunde, eine Vorrichtung und ein Verfahren zur Verfügung zu stellen, welche eine verbesserte Sterilisationswirkung und damit auch einen verbesserten Reinheitsgrad der Anlage erlauben. Dies ist erfindungsgemäß durch eine Vorrichtung nach Anspruch 1 und ein Verfahren nach Anspruch 11 erreicht. Vorteilhafte Ausführungsformen und Weiterbildungen sind Gegenstand der Unteransprüche.

Eine erfindungsgemäße Vorrichtung wird im Anspruch 1 definiert.

Unter einer Reinigungseinrichtung wird dabei eine Einrichtung verstanden, die im weitesten Sinne zum Reinigen eines anderen Elements bzw. Körpers dient. Insbesondere kann es sich bei der Reinigungseinrichtung um eine Sterilisationseinrichtung handeln, welche den betreffenden Körper oder Teile desselben sterilisiert. Denkbar wäre jedoch auch eine beispielsweise mechanisch wirkende Reinigungseinrichtung, welche den Körper nur reinigt, nicht jedoch sterilisiert oder entkeimt. Im Folgenden wird neben dem Begriff der Reinigungseinrichtung auch der Begriff der Sterilisationseinrichtung verwendet. Bevorzugt dient damit die Reinigungseinrichtung zum Sterilisieren der Haltelemente.

Es wird damit erfindungsgemäß vorgeschlagen, dass speziell die Haltelemente für die Behältnisse bzw. Vorformlinge sterilisiert werden. Bei diesen Haltelementen kann es sich um jegliche Halteelemente handeln, welche die Behältnisse kontaktieren, um diese zu transportieren wie beispielsweise Greifklammern und insbesondere aber auch Dome, welche in eine Mündung der Behältnisse eingreifen. Vorzugsweise werden zumindest diejenigen Bereiche der Haltelemente sterilisiert, die anschließend mit den Behältnissen bzw. Vorformlingen in Kontakt sind.

Vorteilhaft ist die Reinigungseinrichtung stationär und besonders bevorzugt in einem Bereich des Transportpfades der Behältnisse angeordnet.

Bei einer weiteren vorteilhaften Ausführungsform weist die Vorrichtung einen Übemahmebereich auf, in dem sie die Behältnisse übernimmt und einen Übergabebereich, in dem sie die Behältnisse abgibt. So ist es beispielsweise möglich, dass die Transporteinrichtung als Transportkette ausgeführt ist, welche die Behältnisse von einem Übergaberad übernimmt und an ein weiteres Sternrad oder auch eine Blasformmaschine weitergibt.

Bei einer weiteren vorteilhaften Ausführungsform ist die Reinigungseinrichtung in einem Bereich des Transportpfads angeordnet, in dem im Arbeitsbetrieb der Vorrichtung keine Behältnisse angeordnet sind. Vorteilhaft ist damit die Reinigungseinrichtung in der Transportrichtung der Haltelemente zwischen dem Übergabebereich und dem Übernahmebereich angeordnet wobei in diesem Bereich keine Behältnisse geführt werden. Auf diese Weise ist eine besonders effiziente Sterilisation der Halteelemente selbst möglich.

Der Transportpfad, auf dem die Halteelemente geführt werden, ist vorzugsweise ein geschlossener Pfad wie er beispielsweise erreicht werden kann, wenn es sich bei der Transporteinrichtung um eine umlaufende Transportkette oder auch um ein Sternrad handelt.

Bei einer weiteren vorteilhaften Ausführungsform weist die Vorrichtung wenigstens ein Heizelement auf um die Behältnisse entlang des Transportpfades zu erwärmen. Wie oben erwähnt, werden bei den im Stand der Technik bekannten Blasformungsvorgängen zunächst die Vorformlinge erwärmt und dann in einem erwärmten Zustand zu Kunststoffbehältnissen expandiert. Zu diesem Zweck sind aus dem Stand der Technik Durchlauföfen bekannt, innerhalb derer die Kunststoffvorformlinge erwärmt werden. In dieser bevorzugten Ausführungsform wird vorgeschlagen, die Reinigungseinrichtung in einem Bereich eines derartigen Ofens anzuordnen. Dies bietet den Vorteil, dass bedingt durch den Erwärmungsvorgang auch die Halteelemente eine höhere Temperatur als Raumtemperatur aufweisen und bedingt durch diese höhere Temperatur auch eine Sterilisierung der Halteelemente in verbesserter Weise möglich ist. Es wäre jedoch auch möglich, die Reinigungseinrichtung auf anderen Transporteinrichtungen, wie beispielsweise Übergabesternen oder dergleichen, anzuordnen.

Bei den Heizelementen kann es sich beispielsweise um Infrarotheizungen oder dergleichen handeln. Es wäre jedoch auch der Einsatz von Mikrowellenheizungen denkbar.

Bei einer weiteren vorteilhaften Ausführungsform weist die Reinigungseinrichtung ein Behältnis mit einer daran angeordneten Sterilisationsflüssigkeit auf. Bei dieser Sterilisationsflüssigkeit kann es sich beispielsweise um Wasserstoffperoxyd (H₂O₂), um Alkohol, Lauge, Schaum, Peressigsäure, oder heißes Wasser oder ähnliche Flüssigkeiten mit sterilisierender Wirkung handeln. In diese Flüssigkeit werden die Heizelemente, beispielsweise die Dorne bzw. Heizdorne auf denen die Behältnisse angeordnet sind, zumindest kurzfristig eingetaucht. Bei einer weiteren vorteilhaften Ausführungsform sind die Halteelemente in diese Sterilisationsflüssigkeit eintauchbar.

Dabei sind besonders bevorzugt die Halteelemente insbesondere in dem Bereich der Reinigungseinrichtung senkrecht zu ihrer Transporteinrichtung bewegbar wobei es ausreicht, wenn zumindest eine senkrechte Komponente vorhanden ist, und beispielsweise die Halteelemente schräg bewegt werden. Ebenso wäre es jedoch auch möglich, dass im Bereich der Reinigungseinrichtung Düsen vorgesehen sind, welche die Haltelemente mit Sterilmedium benetzen. Auch könnten im Bereich der Reinigungseinrichtung andere Reinigungseinrichtungen wie beispielsweise Elektronenstrahlemitter oder auch UV-Strahler angeordnet sein. Auch könnten mehrere unterschiedliche Reinigungseinrichtungen wie Düsen oder Tauchbäder vorgesehen sein.

Durch die erfindungsgemäße Vorrichtung ist eine sterile Verarbeitung von Kunststoffvorformlingen -insbesondere in einem Heizmodul und insbesondere im laufenden Betrieb- möglich. Auf diese Weise kann eine permanente Sterilität der Vorformlingsaufnahme im Heizmodul gewährleistet werden. Vorzugsweise erfolgt die Reinigung kurz nachdem der Kunststoffvorformling an eine weitere Einrichtung wie eine Blasmaschine übergeben wurde und kurz bevor ein neuer Kunststoffvorformling von einem Ofeneinlauf abgeholt wird. Vorzugsweise weisen die Halteelemente Haltedorne auf, welche in die Mündungen der Behältnisse eingreifen. Vorzugsweise sind diese Halteelemente an einer Transportkette angeordnet oder auch wie oben erwähnt an einem Transportstem. Die Sterilisation kann jedoch auch bei anderen Einrichtungen als dem Ofen vorgesehen sein.

Bei einer weiteren vorteilhaften Ausführungsform weist die Reinigunseinrichtung wenigstens eine Bürsteneinrichtung zum Reinigen der Haltelemente auf. Dabei kann es sich beispielsweise um eine drehbare Bürsteneinrichtung handeln, gegenüber der das Halteelement abrollt. Auch wäre es denkbar, dass zwei gegenüberliegende Bürsteneinrichtungen vorgesehen sind, zwischen denen das Haltelement hindurchgeführt wird. Auch wäre es möglich, dass ein oder mehrere Bürstenelemente auf die Halteelemente zugestellt werden.

Bei einer weiteren vorteilhaften Ausführungsform ist in der Transportrichtung der Halteelemente nach der Reinigungseinrichtung eine Trocknungseinrichtung zum Trocken der Halteelemente angeordnet. Vorzugsweise ist diese Trocknungseinrichtung in einem Bereich angeordnet, in dem noch keine neuen Behältnisse aufgenommen wurden. So ist es möglich, dass das Halteelement bzw. die Vorformlingsaufnahme in einen Behälter mit einem sterilen Medium eingetaucht wird, diesen dann für einige Sekunden durchläuft und nach dem Auftauchen mit steriler Luft oder Gas getrocknet wird. Auf diese Weise wird verhindert, dass Rückstände von der Flüssigkeit in den Kunststoffvorformling gelangen.

Weiterhin wäre es, wie oben erwähnt, auch möglich, das Haltelement mit einem sterilen Medium zu besprühen, wobei sich das Halteelement vorteilhaft kontinuierlich weiterdreht, um so eine gleichmäßige Verteilung zu ermöglichen. Anschließend kann die Vorformlingsaufnahme bzw. das Haltelement wieder mit steriler Luft oder mit einem Gas getrocknet werden um Rückstände zu entfernen.

Vorzugsweise sind damit die erwähnten Dorne zur Aufnahme der Kunststoffvorformlinge drehbar angeordnet. Diese drehbare Anordnung ist an sich aus dem Stand der Technik bekannt, um eine vollumfängliche Erwärmung der Kunststoffvorformlinge zu erreichen. Die Drehung wird jedoch hier auch in dem Bereich der Sterilisations- und insbesondere auch in den Bereich der Trocknungseinrichtung fortgeführt.

Weiterhin wäre es auch möglich, die Heizdorne direkt mit Hilfe der Heizstrecke der Vorrichtung zu trocken. So könnte ein Leerdurchlauf vorgesehen sein, in dem die Heizdorne durch die Heizstrecke für die Trocknung laufen bei der beispielsweise Infrarotlampen der Heizeinrichtung eingeschaltet bleiben.

Mit dieser Vorgehensweise könnten auch Abschirmplatten, welche insbesondere die Gewinde der Kunststoffvorformlinge schützen, steril gehalten werden.

Die vorliegende Erfindung ist weiterhin auf eine Anlage zum Behandeln von Behältnissen gerichtet, welche eine Vorrichtung der oben beschriebenen Art aufweist und eine Umformungseinheit zum Umformen der Kunststoffvorformlinge zu Kunststoffflaschen. Vorteilhaft ist dabei die Umformungseinheit in der Transportrichtung der Behältnisse nach der erwähnten Vorrichtung angeordnet.

Bei einer weiteren vorteilhaften Ausführungsform weist die Anlage weitere Reinigungseinrichtungen auf, welche die Behältnisse selbst sterilisieren und insbesondere solche Reinigungseinrichtungen, welche eine Innenwandung der besagten Behältnisse sterilisieren.

Die vorliegende Erfindung ist weiterhin ein Verfahren zum Behandeln von Behältnissen gerichtet, wobei die Behältnisse mit einer Transporteinrichtung entlang eines vorgegebenen Transportpfades transportiert werden wobei die Behältnisse vereinzelt mit einer Vielzahl von an einer Transporteinrichtung angeordneten Haltelementen transportiert werden.

Dabei werden erfindungsgemäß die Halteelemente entlang eines Teilabschnitts des Transportpfads wenigstens abschnittsweise sterilisiert.

Vorteilhaft werden die Halteelemente in einem Abschnitt sterilisiert, in denen kein Behältnis an ihnen angeordnet ist.

In einem weiteren vorteilhaften Verfahren werden die Behältnisse in einem Übernahmebereich von der Transporteinrichtung aufgenommen und in einem Übergabebereich von der Transporteinrichtung abgegeben.

Bei einer weiteren vorteilhaften Ausführungsform werden die Halteelemente nach dem Sterilisieren getrocknet. Dabei ist es möglich, dass die Halteelemente zunächst in ein Tauchbad gesenkt oder besprüht und anschließend getrocknet werden.

Vorteilhaft werden die Behältnisse entlang ihres Transportpfades erwärmt.

Weitere Vorteile und Ausführungsformen ergeben sich aus den beigefügten Zeichnungen.

Darin zeigen:
- Fig. 1: eine schematische Darstellung einer erfindungsgemäßen Anlage;
- Fig. 2: eine schematische Darstellung einer erfindungsgemäßen Vorrichtung;
- Fig. 3: eine Detailansicht der Vorrichtung aus Fig. 2;
- Fig. 4: eine Detaildarstellung einer erfindungsgemäßen Vorrichtung;
- Fig. 5: eine weitere Detaildarstellung einer erfindungsgemäßen Vorrichtung;
- Fig. 6: eine Darstellung einer weiteren Ausführungsform einer erfindungsgemäßen Vorrichtung;
- Fig. 7: drei Schnittdarstellungen zur Veranschaulichung einer Innensterilisation;
- Fig. 8: eine Darstellung einer weiteren Ausführungsform einer erfindungsgemäßen Vorrichtung;
- Fig. 9: eine Detaildarstellung der Vorrichtung aus Fig. 8;
- Fig. 10: eine weitere Ausführungsform einer erfindungsgemäßen Vorrichtung;
- Fig. 11: eine weitere Ausführungsform einer erfindungsgemäßen Vorrichtung;
- Fig. 12: eine weitere Ausführungsform einer erfindungsgemäßen Vorrichtung;
- Fig. 13: eine Darstellung eines Halteelementes;
- Fig. 14: eine Darstellung eines Halteelementes in einer weiteren Ausführungsform;
- Fig. 15: eine Schnittdarstellung des in Fig. 14 gezeigten Halteelementes;
- Fig. 16a,b: zwei Darstellungen einer weiteren Ausführungsform der vorliegenden Erfindung;
- Fig. 17a,b: zwei Darstellungen einer weiteren Ausführungsform der vorliegenden Erfindung, und;
- Fig. 18: eine weitere Darstellung zur Veranschaulichung der in den Fig 17a,b gezeigten Ausführungsformen.

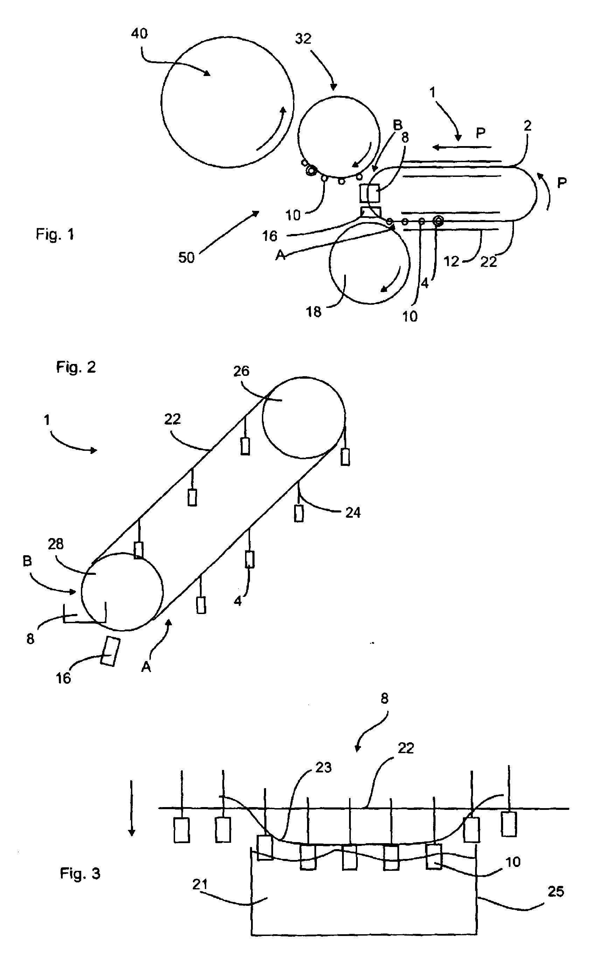

Fig. 1 zeigt eine erfindungsgemäße Anlage zum Herstellen von Kunststoffbehältnissen. Diese Anlage weist dabei eine Vorrichtung 1 wie eine Heizeinrichtung auf, welche Kunststoffvorformlinge 10 erwärmt. Dabei werden die Kunststoffvorformlinge mit Hilfe einer Transporteinrichtung 2 entlang eines Transportpfades P transportiert und in diesem Bereich von Heizelementen 12 erwärmt. Die anschließend erwärmten Behältnisse werden an einen Übergabestem 32 übergeben und von diesem wiederum an eine Umformungseinrichtung 40. Diese Umformungseinrichtung 40 weist dabei eine Vielzahl von (nicht gezeigten) Blasstationen auf, welche die Kunststoffvorformlinge zu Kunststoffbehältnissen expandiert.

Das Bezugszeichen 4 kennzeichnet ein nur schematisch dargestelltes Halteelement, welches jeweils einen Kunststoffvorformling 10 hält bzw. trägt so dass dieser transportiert werden kann. Die Kunststoffvorformlinge 10 werden an die Vorrichtung 1 von einem Übergabestern 18 übergeben.

Das Bezugszeichen A bezieht sich auf einen Übernahmebereich, in dem Kunststoffvorformlinge von dem Transportstern 18 an die Vorrichtung 1 übergeben werden. Das Bezugszeichen B kennzeichnet einen Übergabebereich, in dem die Kunststoffvorformling von der Transportvorrichtung 2 an den Transportstern 32 übergeben werden. Zwischen diesem Übergabebereich B und dem Übernahmebereich A ist in der Transportrichtung der Halteelemente 4, welche hier entgegen dem Uhrzeigersinn verläuft, eine Reinigungseinrichtung 8 vorgesehen, welche die Halteelemente sterilisiert. Nach dieser Reinigungseinrichtung 8 ist eine Trocknungseinrichtung 16 vorgesehen, welche die sterilisierten Halteelemente 4 trocknet.

Fig. 2 zeigt eine erfindungsgemäße Vorrichtung 1. Bei dieser Ausführungsform weist die Transporteinrichtung 2 eine um zwei Umlenkräder 26, 28 umlaufende Transportkette 22 auf, an der eine Vielzahl von Halteelementen 4 angeordnet sind. Diese Halteelemente 4 sind hier als Dorne bzw. Heizdorne ausgeführt, welche in die Behältnisse ragen und welche wiederum an Trägerstangen 24, die an der Transportkette 22 angeordnet sind, befestigt sind. Nach dem Übergabebereich d.h., nach dem die Halteelemente 4 die Behältnisse (nicht gezeigt) abgegeben haben, werden sie durch eine Reinigungseinrichtung 8 geführt, welche hier stationär angeordnet ist und in der ein Reinigungsmedium, insbesondere eine Flüssigkeit wie Wasserstoffperoxyd, angeordnet ist. An die Reinigungseinrichtung 8 schließt sich die Trocknungseinrichtung 16 an.

Fig. 3 zeigt eine mögliche Ausführungsform einer erfindungsgemäßen Reinigungseinrichtung 8. Dabei weist diese Reinigungseinrichtung 8 hier ein Behältnis 25 auf, in welches die einzelnen Halteelemente 4 eingetaucht werden. Zu diesem Zweck ist eine Führungskurve 23 vorgesehen, welche die Halteelemente 4 im Bereich des Behältnisses 25 entlang des Pfeils y nach unten drückt und damit in die Flüssigkeit 21 eintaucht. Dadurch bewirkt diese Führungskurve, dass nach dem Eintauchen die Halteelemente wieder nach oben gelangen. Zu diesem Zweck können an den Halteelementen 4 bzw. deren Trägerstangen 24 Federungseinrichtungen vorgesehen sein, welche die Halteelemente 4 wieder nach oben drängen. Zusätzlich oder daneben können, wie oben erwähnt, auch Reinigungsdüsen vorgesehen sein, welche die einzelnen Haltelemente 4 mit einem Sterilisationsmedium besprühen.

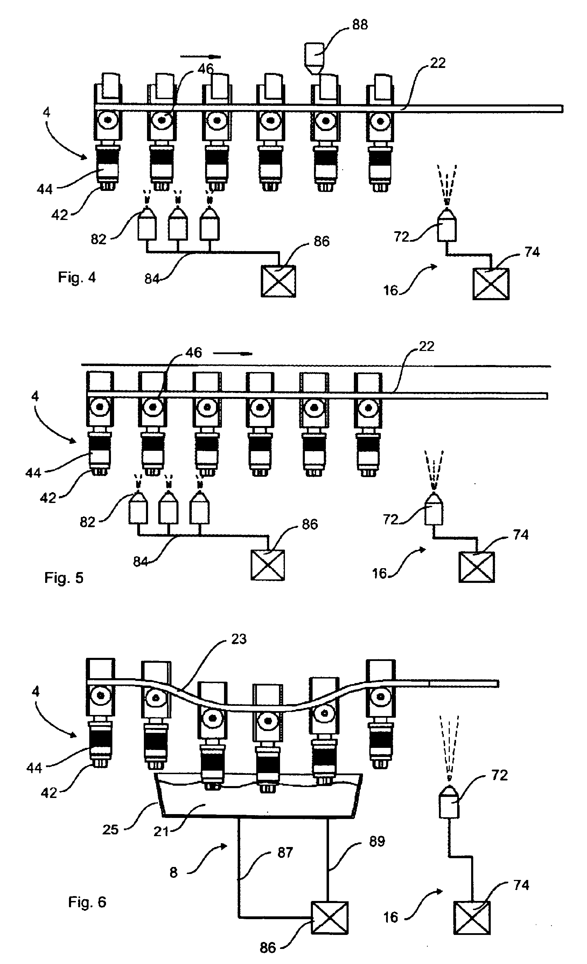

Fig. 4 zeigt eine weitere Detaildarstellung einer erfindungsgemäßen Vorrichtung. Bei dieser Ausführungsform werden die einzelnen Halteelemente 4 in einer horizontalen Richtung gefördert. Die Halteelemente weisen hier einen Dorn 42 auf, der in eine Mündung der Behältnisse (nicht gezeigt) eingreift und der an einem Träger 44 angeordnet ist. Bei dieser Ausführungsform werden die Halteelemente 4 mit einer Vielzahl von Düsen 82 besprüht und auf diese Weise sterilisiert. Zusätzlich ist eine weitere Düse 88 zur Innensterilisation vorgesehen. Das Bezugszeichen 84 bezieht sich auf eine Zuführleitung, um ein Sterilisationsmedium den einzelnen Düsen 82 zuzuführen und das Bezugszeichen 86 auf ein Reservoir für die Flüssigkeit. Damit werden hier die einzelnen Heizdorne 42 mit einem sterilen Mittel eingesprüht, wobei vorteilhaft die Heizdorne vorher in Rotation versetzt bzw. während des Sprühvorgangs rotiert werden. Anschließend werden die Heizdorne 42 mit einem Sterilluftgebläse 72, welches ebenfalls mit einem Reservoir 74 verbunden ist und insgesamt eine Trocknungseinrichtung 16 darstellt getrocknet.

Bei der in Fig. 5 gezeigten Ausführungsform wurde auf die Düse 88 zur Innenreinigung der Halteelemente 4 verzichtet. Das Bezugszeichen 46 bezieht sich auf eine Kurvenrolle, welche an den Halteelementen 4 angeordnet ist und welche zur Führung gegenüber einer Kurve 22 dient.

Fig. 6 zeigt eine detailliertere Darstellung einer erfindungsgemäßen Vorrichtung. Hier werden, wie bereits in Fig. 3 gezeigt, die Halteelemente 4 durch eine spezielle Führungskurve 23 nach unten gedrückt und die Dorne 42 in eine sterile Flüssigkeit 21 eingetaucht. Das Bezugszeichen 86 kennzeichnet hier wieder ein Reservoir für die Sterilflüssigkeit, wobei hier jedoch ein Zulauf 89 von dem Reservoir in das Behältnis 25 vorgesehen ist sowie ein Rücklauf 87. Auch bei dieser Ausführungsform ist eine Trocknungseinrichtung 16 der oben beschriebenen Art stromabwärts bezüglich der Reinigungseinrichtung 8 vorgesehen.

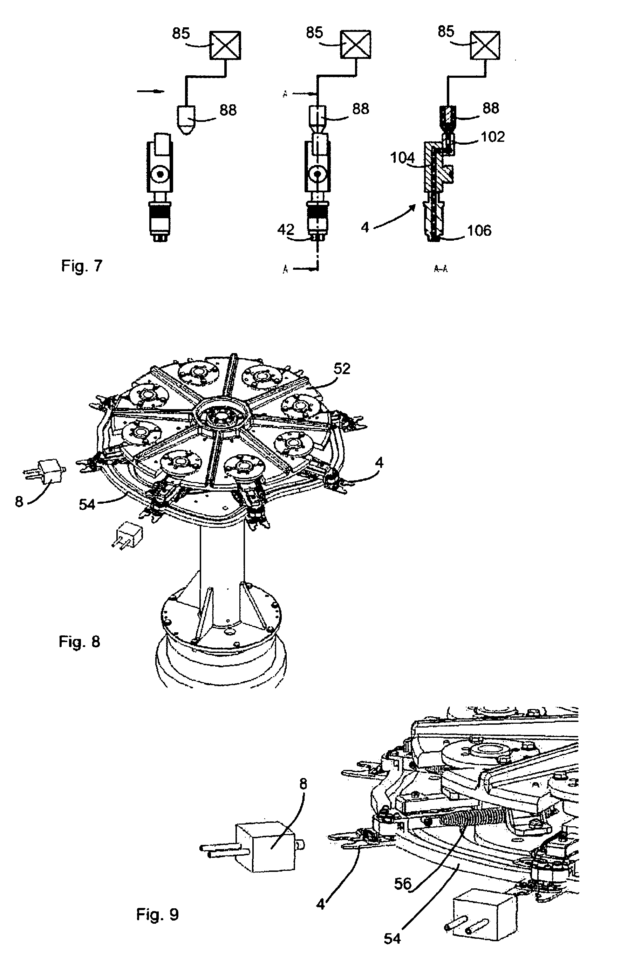

Fig. 7 zeigt eine vereinfachte Darstellung zur Veranschaulichung der Innensterilisation. Die Düsen 88 sind hier zusätzlich mit einem Ventil (nicht gezeigt) ausgestattet, welches sich beim Passieren der Heizdorne öffnet. Bei Öffnen dieses Ventils kann Sterilflüssigkeit über einen Kanal im Inneren des Heizdornes beispielsweise bis zu den Klemmbacken gelangen und somit die Stellen sterilisieren mit denen der Vorformling später in Kontakt kommt.

Bei der linken Darstellung in Fig. 7 ist eine Situation vor dem Kontakt gezeigt, wobei das Ventil, welches die sterile Flüssigkeit zuführt, geschlossen ist. Bei der mittleren Darstellung gelangt die Düse 88 in Kontakt mit einem Lagerklotz 102 der hier seitlich an dem Halteelement 4 angeordnet ist und ein Öffnen des (nicht gezeigten) Ventils bewirkt. Ein Sterilmittel wird auf diese Weise freigesetzt und strömt durch das Halteelement. Die rechte Darstellung von Fig. 7 veranschaulicht einen Schnitt entlang der Linien A-A aus der mittleren Figur. Man erkennt hier im Inneren des Halteelements 4 einen Kanal 104 zum Transport des Sterilmediums in Richtung des Dorn 42. Weiterhin sind (nicht im Detail gezeigt) radial verlaufende Kanäle 106 vorgesehen, welche die Sterilflüssigkeit an die Außenseite des Heizdoms 42 fördern.

Die Fig. 8-12 zeigen eine weitere Ausführungsform einer erfindungsgemäßen Vorrichtung, wobei es sich hier bei den Halteelementen 4 um Greifklammern handelt, welche das Behältnis an seinen Außenumfang, beispielsweise unterhalb eines Tragrings, greifen. Das Bezugszeichen 52 kennzeichnet ein Trägerrad, an dem eine Vielzahl von Halteelementen 4 angeordnet ist. Auch hier sind radial außerhalb dieser Halteelemente Reinigungseinrichtungen 8 vorgesehen, welche hier ein Sterilmittel in radialer Richtung auf die Halteelemente 4 aufbringen. Wie in Fig. 8 gezeigt können hier mehrere Reinigungseinrichtungen um den Außenumfang angeordnet sein. Vorzugsweise ist auch hier die Reinigungseinrichtung 8 stationär angeordnet.

Fig. 9 zeigt eine Detaildarstellung der in Fig. 8 gezeigten Vorrichtung. Man erkennt hier, dass die Vorrichtung ebenfalls eine Führungskurve 54 aufweist, welche die Halteelemente radial nach außen drängt, wenn diese in Umfangsrichtung der Reinigungseinrichtungen 8 passieren. Ein Federlement 56 bewirkt, dass die Halteelemente in radialer Richtung wieder eingezogen werden und sich somit an den Lauf der Führungskurve 54 anpassen.

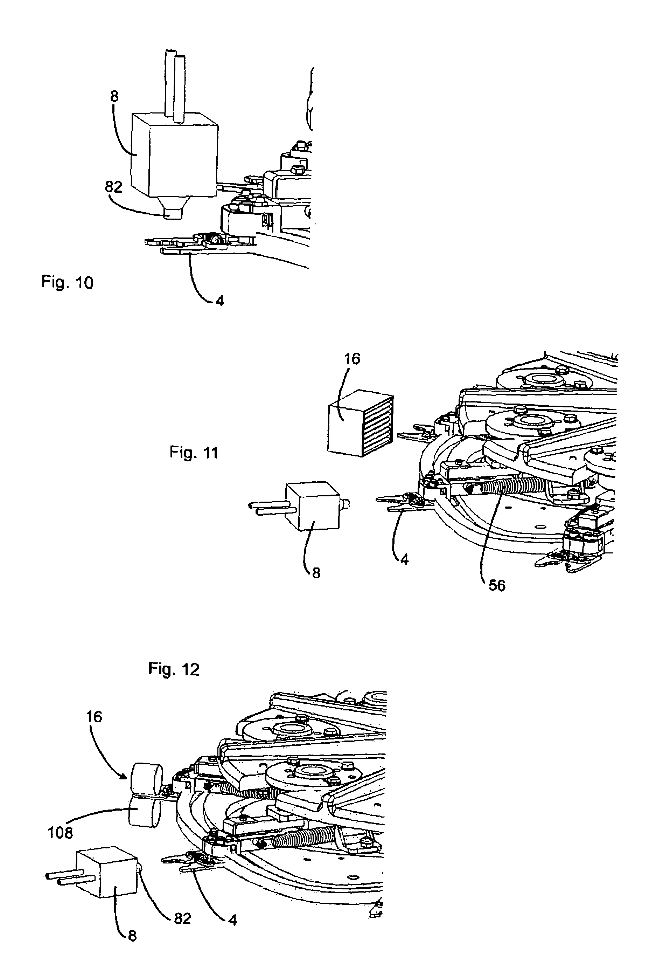

Fig. 10 zeigt eine weitere Ausführungsform einer erfindungsgemäßen Reinigungseinrichtung, wobei hier eine Sterilisation von oben erfolgt. Die Auslösung bzw. Betätigung eines (nicht gezeigten) Ventils dieser Reinigungseinrichtung 8 könnte beispielsweise in Abhängigkeit einer Drehstellung der Halteelemente 4 erfolgen wobei jedoch auch andere Steuerungsmechanismen, wie beispielsweise Lichtschrankensteuerungen, möglich wären.

Fig. 11 zeigt eine weitere Ausführungsform einer erfindungsgemäßen Vorrichtung, wobei hier stromabwärts bezüglich der Reinigungseinrichtung eine Trocknungseinrichtung 16 dargestellt ist, welche beispielsweise die Halteelemente 4 mit Luft beaufschlagt.

Fig. 12 zeigt eine weitere Ausführungsform einer erfindungsgemäßen Vorrichtung. Anstelle einer Trocknungseinrichtung, die als Belüftungseinrichtung ausgeführt ist, weist die Trocknungseinrichtung hier zwei Rollen 108 auf, zwischen denen die Halteelemente 4 durchgeführt werden, und welche zum Trocknen der Halteelemente 4 dient.

Diese Rollen 108 könnten jedoch auch als Bürsten zum Reinigen der Halteelemente ausgeführt sein, oder es könnte ein weiteres (nicht gezeigtes) Rollenpaar vorgesehen sein. Derartige Bürsten könnten dabei insbesondere derart gestaltet sein, dass auch die inneren Bereiche der Halteelemente 4 gereinigt werden.

Es wird darauf hingewiesen, dass die besagten Rollen nicht nur zum Trocknen sondern gegebenenfalls bei entsprechender Beaufschlagung mit einem Sterilisationsmedium auch zur Sterilisation verwendet werden könnten.

Damit können mit der erfindungsgemäßen Vorgehensweise unterschiedlichste Halteelemente sterilisiert werden. Wie bei den Heizdornen, kann auch bei den Greifklammern mit den Düsen Sterilisationsflüssigkeit aufgetragen werden, und ggf. mit einer zweiten Düse diese wieder mit Sterilluft getrocknet werden. Wie erwähnt, kann sich zum Trocknen, aber auch zum Aktivieren der Sterilisationsflüssigkeit ein Infrarotstrahl ereignen. Die hier dargestellte Trocknung und auch das Sterilisieren ist jedoch auch von unterschiedlichen Seiten oder ggf. auch von unten denkbar. Daneben wäre es auch möglich, die Halteelemente 4 mit Bürsten zu säubern. Dabei könnten, wie erwähnt, diese Bürsten bzw. Walzen zusätzlich Sterilisationsflüssigkeit auftragen, in dem sie mit einer Durchleitung von innen versehen werden.

Daneben kann auch ein (nicht gezeigter) Abtropfschutz vorgesehen sein, der bewirkt, dass die Flüssigkeit, das heißt das Sterilisationsmedium, nicht in der ganzen Maschine verteilt wird. Auch dieser Abtropfschutz könnte das Sterilisationsmedium zu einer Aufbereitung zurückführen.

Daneben könnten weiterhin Sensoren, insbesondere optische Sensoren, vorgesehen sein, welche die Halteelemente regelmäßig auf Verschmutzung bzw. Sterilität überprüfen. Eine Reinigung der Halteelemente kann entweder in einem Reinigungszyklus erfolgen, bei dem die Maschine langsamer gefahren werden kann oder auch kontinuierlich im Arbeitsbetrieb. Insbesondere im letzteren Fall findet bevorzugt, wie oben erwähnt, die Sterilisation bzw. Reinigung in einem Bereich statt, in dem kein Vorformling in Eingriff ist, das heißt, bei Heizdornen nach der Übergabe des Vorformlings an die Transportsteme und vor der neuen Aufnahme eines Kunststoffvorformlings. Im Falle von Klammern könnte dies der Bereich nach der Abgabe eines Kunststoffvorformlings an das Blasrad und vor der neuen Aufnahme des Kunststoffvorformlings vom Heizmodul sein.

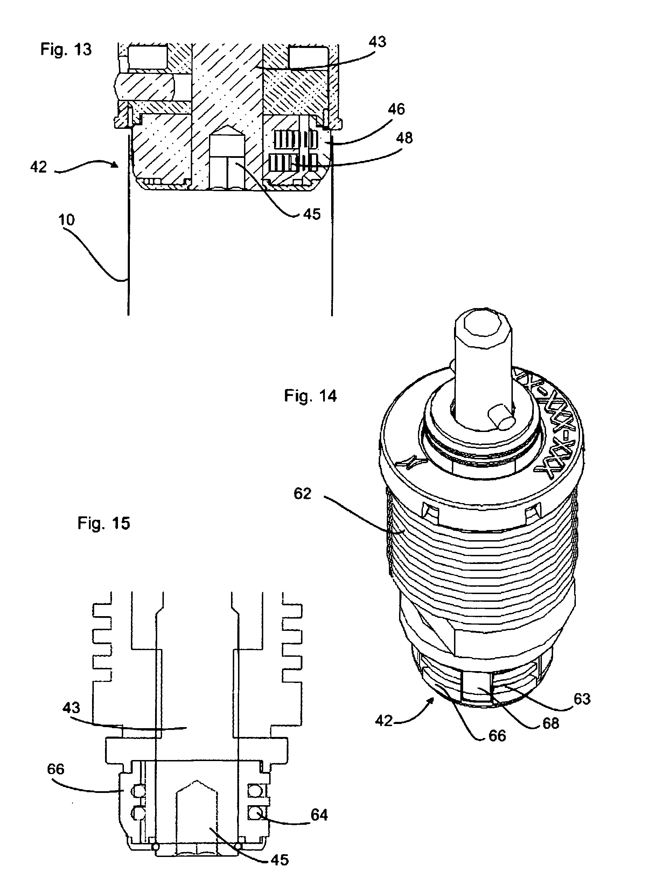

Fig. 13 zeigt eine Darstellung eines Heizdorns 42, an dem ein (nur teilweise und angedeutet dargestellter Kunststoffvorformling 10 gehalten ist. An diesem Heizdorn sind Aufnahmeelemente 46 vorgesehen, welche mit Federungselementen 48 abgefedert bzw. radial nach außen gedrängt werden. Weiterhin ist ein Verbindungsmittel wie eine Schraube 45 vorgesehen, um den Haltedorn 42 von einer Stange 43 abnehmen zu können. Die Federungseinrichtung 48 ist hier vorteilhaft aus einem Material wie V4A (rostfreier Stahl) hergestellt, um Korrisionen widerstehen zu können und um insbesondere Beaufschlagungen mit dem Sterilisationsmittel zu überstehen.

Fig. 14 zeigt eine weitere Ausführungsform eines erfindungsgemäßen Halteelementes. In diesem Falle ist ein Heizdorn 42 mit O-Ringen vorgesehen, wobei diese O-Ringe hier vorteilhaft in Nuten 63 angeordnet sind.

Fig. 15 zeigt eine entsprechende Schnittdarstellung des in Fig. 14 gezeigten Heizdoms. Man erkennt hier insbesondere die einzelnen O-Ringe 64, die vorteilhaft aus Materialien wie EPDM (Ethylen Propylen Dien) -Kautschuk, Perfluor-Kautschuk, wie beispielsweise FFKM oder einem geeigneten Materialverbund gefertigt sind. Daneben wäre es auch möglich, als O-Ringe 64 Teflon-Ringe zu verwenden. Diese O-Ringe sind dabei in den auch in Fig. 14 gezeigten Nuten 63 angeordnet und belasten hier die Aufnahmeelemente 66 radial nach außen. Dies bedeutet, dass die O-Ringe 64 eine Federungswirkung auf die Aufnahmeelemente 66 ausüben. Diese Aufnahmeelemente 66 sind jedoch nicht vollumfänglich angeordnet, sondern zwischen zwei Befestigungsmitteln 66 sind, wie in Fig. 14 gezeigt, auch Aussparungen 68 vorgesehen. Auch in diesem Falle ist der Haltedorn mit Hilfe einer Verbindungseinrichtung 45 an einer Stange 43 angeordnet. Die oben erläuterten Maßnahmen zur Innensterilisation sind hier jedoch nicht dargestellt.

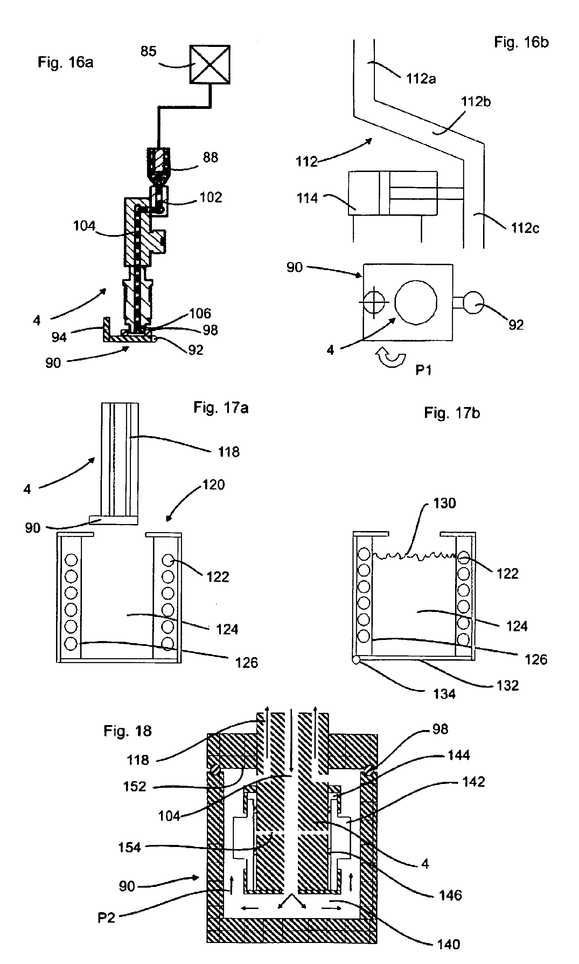

Die Figuren 16a und 16b zeigen eine weitere Ausführungsform der vorliegenden Erfindung. Zusätzlich zu der in Fig. 7 gezeigten Ausführungsform ist hier noch eine Abdeckeinrichtung 90 wie etwa eine sog. CIP - Kappe vorgesehen, welche unterhalb des Halteelements 4 angeordnet ist. Diese Abdeckeinrichtung ist bei dieser Ausführungsform schwenkbar gegenüber dem Halteelement 4 angeordnet. So ist es möglich, dass diese Abdeckeinrichtungen 90 schwenkbar an einer Ofenkette (nicht gezeigt) mitfahrend gelagert sind. Denkbar wäre auch, dass die CIP - Kappen 90 nicht jedem Halteelement 4 zugeordnet sind, sondern stationär angeordnet sind, um ein taktweises Reinigen der Halteelemente 4 zu ermöglichen. Auch ein zeitweises Begleiten der Halteelemente 4 von den CIP - Kappen 90 wäre denkbar. Auf diese Weise wäre auch ein kontinuierlicher Reinigungsvorgang an der Begleitstrecke möglich.

Vorteilhaft ist daher an wenigstens einem Halteelement 4eine Abdeckeinrichtung 90 angeordnet, wobei eine Reinigungsflüssigkeit wenigstens abschnittsweise über diese Abdeckeinrichtung 90 führbar ist.

Das Bezugszeichen 112 in Fig. 16b kennzeichnet entsprechend eine stationär angeordnete Führungskurve, mit der eine Laufrolle 92, welche an der Abdeckeinrichtung 90 angeordnet ist, bewegt werden kann, um die Abdeckeinrichtung (in Fig. 16b in der Figurenebene) zu schwenken. Diese Führungskurve ist dabei zusätzlich mittels einer Antriebseinrichtung wie hier eines Pneumatikzylinders 114 schwenkbar oder ausfahrbar. Dabei ist es möglich, dass ein zweites Kurvensegment 112c gegenüber einem ersten Kurvensegment 112a verschoben werden kann, um die Führungskurve 112 zu verstellen. Das Bezugszeichen 112b bezieht sich auf ein entsprechendes Übergangssegment. Durch die in Fig. 16b schematisch dargestellte Anpassung der Kurve 112 kann ein Schwenkvorgang der Abdeckeinrichtung erreicht werden. Genau so ist es aber auch möglich, die Kurvensegmente 112a, 112b und 112c gemeinsam zuzustellen bzw. zu verfahren.

Das Bezugszeichen 94 in Fig. 16a kennzeichnet eine Anlenkung wie eine Schwenkwelle, um die die Abdeckeinrichtung 90 schwenkbar ist. Das Bezugszeichen 98 kennzeichnet eine Abdichteinrichtung, um das Halteelement (insbesondere während eines Spülvorgangs abzudichten.

Zu Reinigungszwecken läuft ein Reinigungs- oder Sterilsationsmedium hierbei (bevorzugt kontinuierlich) über eine Drehverteilung im Ofen durch den Heizdorn und / oder die Abschirmplatte hindurch und wird von der CIP-Kappe über eine nicht dargestellte Rückführleitung wieder rückgeführt. Denkbar wären auch Servozustellungen für die Kappen.

Bevorzugt werden die Heizdorne auf diese Weise auf einem Rundläuferofen (beispielsweise STIR (selektives transformiertres Infrarot) oder Mikrowelle) sterilisiert. Hier hätte man auch eine leichtere Drehverteilung. Es wäre auch denkbar auf diese Weise die einem oder mehreren Preform zugeordneten Heizkavitäten, also zum Beispiel die Resonatoren oder Heiztaschen zu reinigen/sterilisieren.

Die Fig. 17a und 17b zeigen eine weitere Ausführungsform der vorliegenden Erfindung. Hier sind sog. Heizaufnahmen 120 bzw. Heiztaschen vorgesehen, in denen die Kunststoffvorformlinge während des Arbeitsbetriebs erwärmt werden. Diese Heizaufnahmen bewegen sich dabei mit den Kunststoffvorformlingen mit. Damit fungieren diese Heizaufnahmen 120 als die Vorformlinge umgebende Bestrahlungskammern. Diese Heizaufnahmen können dabei karusselförmig angeordnet sein. Die Bezugszeichen 122 beziehen sich auf Infrarotstrahler, welche die in dem Aufnahmeraum 124 angeordneten Kunststoffvorformlinge erwärmen.

Eine Innenwand der Heizaufnahmen 120 kann dabei als keramischer Infrarotstrahler ausgebildet sein. Daneben ist es möglich, dass ein stabförmiger Infrarotstrahler (nicht gezeigt) in die Kunststoffvorformlinge eingeführt wird um diese zu erwärmen. Vorteilhaft ist dabei der Kunststoffvorformling vollständig (ggfs. bis auf seine Mündung) innerhalb der Heizaufnahme angeordnet. Das Halteelement 4 bzw. der Heizdorn muss selbst jedoch nicht als IR- Strahler ausgebildet sein, sondern etwa nur reflektieren oder den Kunststoffvorformling halten. Das Bezugszeichen 126 kennzeichnet eine Schutzscheibe für die IR - Strahler.

Fig. 17b zeigt die Heizaufnahme 120 in einem Reinigungsbetrieb. Hier wird diese Heizaufnahme durch Anfüllen mit einem Sterilisationsmedium 130 sterilisiert. Genauer können hier auch die Heizstrahler 122 und die Reflektoren gereinigt werden. Das Bezugszeichen 132 bezieht sich auf einen Boden der Heizaufnahme, der hier auch einen Auslass für das Sterilisationsmedium 130 bildet. Zum Öffnen kann der Boden um eine Schwenkachse 134 geschwenkt werden. In Fig. 17a ist zusätzlich auch ein Rückleitungskanal 118 erkennbar, der zum Rückführen des Sterilisationsmediums dient. Die Heizaufnahme 120 kann insbesondere durch den Halteelement 4 selbst befüllt werden. Somit ist eine eigene Abdeckeinrichtung 90 für den Dorn 4 nicht zwingend nötig. Auf diese Weise ist auch ein Eintauchen eines Halteelements 4 zur Reinigung oder Sterilisation der Außenflächen möglich. In anderen Worten könnte so die Heizaufnahme 120 die Funktion der Abdeckeinrichtung 90 übernehmen.

Fig. 18 zeigt eine weitere Darstellung zur Veranschaulichung eines Sterilisationsvorgangs. Über den Kanal 104 wird das Reinigungsmedium in einen von der Abdeckeinrichtung 90 ausgebildeten Aufnahmeraum 140 geleitet. Von dort strömt das Reinigungsmedium wieder nach oben (Pfeil P2) und über den Rückleitungskanal 118 zurück. Zu diesem Zweck wird die Abdeckeinrichtung 90 an einen Anschlag 152 angedrückt, wobei dieser Anschlag im Arbeitsbetrieb der Anlage auch als Anschlag für die Kunststoffvorformlinge dienen kann.

Das Bezugszeichen 142 bezieht sich auf eine Greiferbacke, welche zum Halten der Kunststoffvorformlinge dient. Auch diese Greiferbacken 142 können mit gereinigt werden. Zu diesem Zeck weist das Halteelement einen Teilkanal 154 auf, welcher zum Hinterspülen der Greiferbacken 142 dient. Das Bezugszeichen 98 bezieht sich wieder auf Dichtungseinrichtungen zum Abdichten der Abdeckeinrichtung 90 gegenüber dem Halteelement während des Spülvorgangs. Mittels einer Federungseinrichtung 146 (bei der es sich aber auch um einen elastischen O-Ring handeln kann), wird die Greiferbacke 142 nach aussen vorgespannt. Das Bezugszeichen 144 kennzeichnet einen Halteraum zum Halten der Greiferbacken 142. In anderen Worten wird das Halteelement 4 sowohl von innen als auch von außen gereinigt bzw. sterilisiert.

Die in den Figueren 16 bis 18 gezeigten Abdeckelemente 90 könnten auch zumindest teilweise aus einem reflektierendem Material ausgebildet sein, um während eines Normalbetriebs der Vorrichtung Strahlung, insbesondere auf den Vorformling, zu reflektieren.

Die Reinigungs- oder Sterilisiationsflüssigkeit kann auch zur Kühlung einzelner Elemente wie der Heizdorne im Normalbetrieb Verwendung finden.

Daneben wird darauf hingewiesen, dass die vorliegende Erfindung auch für solche Maschinen Anwendung finden kann, welche die Kunststoffvorformlinge in deren Umfangsrichtung ungleichmäßig erwärmen (preferential heating). Eine derartige Vorrichtung wurde beispielsweise in der noch nicht veröffentlichten Deutschen Patentanmeldung Nr. 10 2009 021 792.4 dargestellt.

Hierbei könnten beispielsweise Klammern gereinigt werden, welche die Kunststoffvorformlinge berühren, um so ein ungleichmäßiges Temperaturprofil auf diese aufzubringen. Die Reinigung könnte hier über Bürsten oder auch über einen CIP - Modus vorgenommen werden.

Daneben könnte auch eine Reinigung eines Sterilisationsmoduls wie in der PCT/EP2009/059923 beschrieben, vorgenommen werden.

### Bezugszeichenliste

- 1: Vorrichtung
- 2: Transporteinrichtung
- 4: Halteelemente
- 8: Reinigungseinrichtung
- 10: Behältnisse
- 12: Heizelement
- 14: Behältnis
- 16: Trocknungseinrichtung
- 18: Übergabestem
- 21: Sterilmedium
- 22: Transportkette
- 23: Führungskurve
- 24: Trägerstange
- 25: Behältnis
- 26, 28: Umlenkrad
- 32: Übergabestern, Transportstern
- 40: Umformungseinrichtung
- 42: Dorn, Heizdorn
- 43: Stange
- 44: Träger
- 45: Schraube
- 46: Aufnahmeelement
- 48: Federungselement
- 50: Anlage
- 52: Trägerrad
- 54: Führungskurve
- 56: Federelement
- 63: Nut
- 64: O-Ring
- 66: Aufnahmeelement
- 68: Aussparung
- 72: Sterilluftgebläse
- 74: Reservoir
- 82: Düse
- 84: Zuführleitung
- 86: Reservoir
- 87: Rücklauf
- 88: Düse
- 89: Zulauf
- 90: Abdeckeinrichtung
- 92: Laufrolle
- 94: Anlenkung
- 98: Abdichteinrichtung
- 102: Lagerklotz
- 104: Kanal
- 106: Kanäle
- 108: Rolle
- 112: Führungskurve
- 112a-c: Segmente der Führungskurve
- 114: Pneumatikzylinder
- 118: Rückleitungskanal
- 120: Heizaufnahme
- 122: Infrarotstrahler
- 124: Aufnahmeraum
- 126: Schutzscheibe
- 130: Sterilisationsmedium
- 132: Boden der Heizaufnahme 120
- 134: Schwenkachse
- 140: Aufnahmeraum
- 142: Greiferbacke
- 144: Halteraum
- 146: Federungseinrichtung
- 152: Anschlag
- 154: Teilkanal

- A: Übernahmebereich
- B: Übergabebereich
- P: Transportpfad
- P1, P2: Pfeile

## Patentansprüche

1. Vorrichtung (1) zum Umformen von Kunststoffvorformlingen zu Kunststoffbehältnissen mit einer Transporteinrichtung (2), welche die Behältnisse (10) entlang eines vorgegebenen Transportpfads (P) transportiert, wobei die Transporteinrichtung (2) eine Vielzahl von Halteelementen (4) zum Halten der Behältnisse (10) aufweist, wobei die Halteelemente (4) entlang des vorgegebenen Transportpfads (P) transportiert werden,
**dadurch gekennzeichnet, dass**
die Vorrichtung eine Reinigungs- und/oder Sterilisationseinrichtung (8) zum Reinigen und/oder Sterilisieren zumindest derjenigen Bereiche der Halteelemente (4), welche anschließend mit den Behältnissen und/oder Vorformlingen in Kontakt sind, aufweist.

2. Vorrichtung nach wenigstens einem der vorangegangenen Ansprüche,
**dadurch gekennzeichnet, dass**
die Reinigungs- und/oder Sterilisationseinrichtung in einem Bereich (C) des Transportpfads (P) angeordnet ist, in dem im Arbeitsbetrieb der Vorrichtung keine Behältnisse angeordnet sind.

3. Vorrichtung (1) nach Anspruch 1,
**dadurch gekennzeichnet, dass**
die Vorrichtung wenigstens ein Heizelement (12) aufweist, um die Behältnisse entlang des Transportpfads (P) zu erwärmen.

4. Vorrichtung (1) nach wenigstens einem der vorangegangenen Ansprüche,
**dadurch gekennzeichnet, dass**
die Reinigungs- und/oder Sterilisationseinrichtung (8) ein Behältnis (14) mit einer darin angeordneten Sterilisationsflüssigkeit aufweist, in welches die Halteelemente (4) eintauchbar sind.

5. Vorrichtung nach einem der Ansprüche 1 bis 3,
**dadurch gekennzeichnet, dass**
die Reinigungs- und/oder Sterilisationseinrichtung (8) mindestens eine Düse zur Benetzung der Halteelemente (4) mit einem Sterilmedium aufweist.

6. Vorrichtung nach wenigstens einem der vorangegangenen Ansprüche,
**dadurch gekennzeichnet, dass**
die Halteelemente (4) Haltedorne (4, 42), welche in Mündungen der Behältnisse (10) eingreifen, oder Greifklammern (4), welche die Behältnisse am Außenumfang greifen, aufweisen.

7. Vorrichtung nach wenigstens einem der vorangegangenen Ansprüche,
**dadurch gekennzeichnet, dass**
die Reinigungs- und/oder Sterilisationseinrichtung wenigstens eine Bürsteneinrichtung zum Reinigen und/oder Sterilisieren der Haltelemente (4) aufweist.

8. Vorrichtung (1) nach wenigstens einem der vorangegangenen Ansprüche,
**dadurch gekennzeichnet, dass**
in der Transportrichtung der Halteelemente (4) nach der Reinigungs- und/oder Sterilisationseinrichtung (8) eine Trocknungseinrichtung (16) zum Trocknen der Halteelemente angeordnet ist.

9. Vorrichtung (1) nach wenigstens einem der vorangegangenen Ansprüche,
**dadurch gekennzeichnet, dass**
die Transporteinrichtung (2) ein Sternrad ist.

10. Anlage (50) zum Behandeln von Behältnissen mit einer Vorrichtung (1) nach wenigstens einem der vorangegangenen Ansprüche und mit einer Umformungseinheit zum Umformen von Kunststoffvorformlingen zu Kunststoffflaschen.

11. Anlage (50) gemäß Anspruch 10,
**dadurch gekennzeichnet, dass**
die Anlage (50) zumindest eine weitere Sterilisationsvorrichtung zur Sterilisation der Behältnisse aufweist.

12. Verfahren zum Umformen von Kunststoffvorformlingen zu Kunststoffbehältnissen, wobei die Behältnisse mit einer Transporteinrichtung (2) entlang eines vorgegebenen Transportpfads (P) transportiert werden, wobei die Behältnisse vereinzelt mit einer Vielzahl an der Transporteinrichtung angeordneten Halteelementen (8) transportiert werden,
**dadurch gekennzeichnet, dass**
zumindest diejenigen Bereiche der Haltelemente (8), welche anschließend mit den Behältnissen und/oder Vorformlingen in Kontakt sind, entlang eines Teilabschnitts des Transportpfads gereinigt und/oder sterilisiert werden.

13. Verfahren nach Anspruch 12,
**dadurch gekennzeichnet, dass**
die Halteelemente (8) in einem Abschnitt gereinigt und/oder sterilisiert werden, in denen kein Behältnis an ihnen angeordnet ist.

14. Verfahren nach wenigstens einem der Ansprüche 12 oder 13,
**dadurch gekennzeichnet, dass**
die Reinigung und/oder Sterilisation der Halteelemente (4) in einem Reinigungszyklus erfolgt, bei dem die Maschine langsamer gefahren werden kann, wobei sich die Halteelemente (4) während der Reinigung und/oder Sterilisation kontinuierlich weiterdrehen.

15. Verfahren nach wenigstens einem der Ansprüche 12 bis 14,
**dadurch gekennzeichnet, dass**
die Halteelemente (4) nach dem Reinigen und/oder Sterilisieren getrocknet werden.

16. Verfahren nach wenigstens einem der Ansprüche 12 bis 15,
**dadurch gekennzeichnet, dass**
ein zur Reinigung und/oder Sterilisation der Halteelemente (4) verwendetes Sterilisationsmodul selbst gereinigt und/oder sterilisiert wird.

## Claims

1. An apparatus (1) for moulding plastic preforms into plastic containers, including a transport device (2) which transports the containers (10) along a specified transport path (P), the transport device (2) having a plurality of holding elements (4) for holding the containers (10), with the holding elements (4) being transported along the specified transport path (P),
**characterised in that** the apparatus includes a cleaning and/or sterilising device (8) for cleaning and/or sterilising the holding elements (4) at least the in sections, which are to be in contact with the containers or performs subsequently.

2. The apparatus as claimed in at least one of the preceding claims, **characterised in that** the cleaning device is disposed in a zone (C) of the transport path (P), in which no containers are present during working operation of the apparatus.

3. The apparatus (1) as claimed in at least one of the preceding claims, **characterised in that** the apparatus has at least one heating element (12) for heating the containers along the transport path (P).

4. The apparatus (1) as claimed in at least one of the preceding claims, **characterised in that** the cleaning and/or sterilising device (8) includes a container (14) having a sterilisation liquid provided therein, into which the containers can be immersed.

5. The apparatus (1) as claimed in one of claims 1 to 3
**characterised in that**
the cleaning or sterilising device comprises at least one mandrel for wettering the holding elements (4) with a sterile mechanism.

6. The apparatus as claimed in at least one of the preceding claims, **characterised in that** the holding elements include holding mandrels (22) which engage in the mouth of the containers (10), or brackets, which grip the containers at their outer XXX.

7. The apparatus as claimed in at least one of the preceding claims, **characterised in that** the cleaning device includes at least one brushing means for cleaning and/or sterilising the holding elements (4).

8. The apparatus (1) as claimed in at least one of the preceding claims, **characterised in that** a drying device (16) for drying the holding elements is provided in the transport direction of the holding elements (4) downstream of the cleaning and/or sterilising device (8).

9. The apparatus (1) as claimed in at least one of the preceding claims
**characterised in that**
the transport device is a star wheel.

10. A system (50) for treating containers using an apparatus (1) as claimed in at least one of the preceding claims and including a moulding unit for moulding plastic preforms into plastic bottles.

11. A system as claimed in claim 10
**characterised in that**
the system comprises a further sterilisation device for sterilising the containers.

12. A method for moulding plastic preforms into plastic containers, wherein the containers are transported by means of a transport device (2) along a specified transport path (P), wherein the containers are transported in an isolated manner by a plurality of holding elements (8) arranged on the transport device,
**characterised in that** the holding elements (8) are sterilised along a partial section of the transport path at least in those sections, which are to be in contact with the containers or preforms subsequently.

13. The method as claimed in Claim 11, **characterised in that** the holding elements (8) are sterilised in a section in which they have no containers arranged thereon.

14. The method as claimed in at least one of claims 12 or 13
**characterised in that**
the cleaning and/or sterilisation of the holding elements (4) takes place in a cleaning cycle in which the machine can be operated slower, wherein the holding elements (4) rotate continuously during cleaning and/or sterilisation.

15. The method as claimed in at least one of the preceding claims, **characterised in that** the holding elements are dried after cleaning and/or sterilisation.

16. The method as claimed in at least one of claims 12-15
**characterised in that**
a sterilising module used for cleaning and/or sterilising of the holding elements is itself cleaned and/or sterilised.

## Revendications

1. Dispositif (1) pour la transformation d'ébauches plastique en récipients plastique comprenant un système de transport (2), lequel transporte les récipients (10) le long d'un chemin de transport (P) prédéfini, le système de transport (2) présentant une pluralité d'éléments de retenue (4) pour la retenue des récipients (10), les éléments de retenue (4) étant transportés le long du chemin de transport (P) prédéfini,
**caractérisé en ce que**
le dispositif présente un système de nettoyage et/ou de stérilisation (8) pour le nettoyage et/ou la stérilisation au moins des zones des éléments de retenue (4), qui sont en contact ensuite avec les récipients et/ou ébauches.

2. Dispositif selon la revendication précédente,
**caractérisé en ce que**
le système de nettoyage et/ou de stérilisation est disposé dans une zone (C) du chemin de transport (P), dans laquelle aucun récipient n'est disposé lorsque le dispositif est en mode de travail.

3. Dispositif (1) selon la revendication 1,
**caractérisé en ce que**
le dispositif présente un élément de chauffage (12), pour réchauffer les récipients le long du chemin de transport (P).

4. Dispositif (1) selon au moins l'une des revendications précédentes,
**caractérisé en ce que**
le système de nettoyage et/ou de stérilisation (8) présente un récipient (14) avec un liquide de stérilisation disposé à l'intérieur, dans lequel les éléments de retenue (4) peuvent être immergés.

5. Dispositif selon l'une des revendications 1 à 3,
**caractérisé en ce que**
le système de nettoyage et/ou de stérilisation (8) présente au moins une buse pour l'humectation des éléments de retenue (4) avec un fluide stérile.

6. Dispositif selon au moins l'une des revendications précédentes,
**caractérisé en ce que**
les éléments de retenue (4) présentent des mandrins de retenue (4, 42), qui s'engagent dans des orifices des récipients (10), ou des pinces de préhension (4), qui saisissent les récipients sur le pourtour extérieur.

7. Dispositif selon au moins l'une des revendications précédentes,
**caractérisé en ce que**
le dispositif de nettoyage et/ou de stérilisation présente au moins un système de brosse pour le nettoyage et/ou la stérilisation des éléments de retenue (4).

8. Dispositif (1) selon au moins l'une des revendications précédentes,
**caractérisé en ce que**
un système de séchage (16) destiné au séchage des éléments de retenue est disposé dans le sens de transport des éléments de retenue (4) en aval du système de nettoyage et/ou de stérilisation (8).

9. Dispositif (1) selon au moins l'une des revendications précédentes,
**caractérisé en ce que**
le système de transport (2) est une roue-étoile.

10. Installation (50) pour le traitement de récipients comprenant un dispositif (1) selon au moins l'une des revendications précédentes et une unité de transformation pour la transformation d'ébauches plastique en flacons plastique.

11. Installation (50) selon la revendication 10,
**caractérisée en ce que**
l'installation (50) présente au moins un autre dispositif de stérilisation pour la stérilisation des récipients.

12. Procédé pour la transformation d'ébauches plastique en récipients plastique, les récipients étant transportés avec un système de transport (2) le long d'un chemin de transport (P) prédéfini, les récipients étant transportés de façon individuelle avec une pluralité d'éléments de retenue (8) disposés sur le système de transport,
**caractérisé en ce que**
au moins les zones des éléments de retenue (8), qui sont en contact ensuite avec les récipients et/ou les ébauches, sont nettoyées et/ou stérilisées le long d'un tronçon du chemin de transport.

13. Procédé selon la revendication 12,
**caractérisé en ce que**
les éléments de retenue (8) sont nettoyés et/ou stérilisés dans un tronçon, dans lequel aucun récipient n'est disposé sur eux.

14. Procédé selon au moins l'une des revendications 12 ou 13,
**caractérisé en ce que**
le nettoyage et/ou la stérilisation des éléments de retenue (4) s'effectue au cours d'un cycle de nettoyage, durant lequel la machine peut être ralentie, les éléments de retenue (4) continuant de tourner en continu pendant le nettoyage et/ou la stérilisation.

15. Procédé selon au moins l'une des revendications 12 à 14,
**caractérisé en ce que**
les éléments de retenue (4) sont séchés après le nettoyage et/ou la stérilisation.

16. Procédé selon au moins l'une des revendications 12 à 15,
**caractérisé en ce que**
un module de stérilisation utilisé pour le nettoyage et/ou la stérilisation des éléments de retenue (4) est autonettoyé et/ou autostérilisé.
